(19) Europäisches Patentamt
European Patent Office
Office européen des brevets

(11) Numéro de publication: **0 233 106**
**B1**

# (12) FASCICULE DE BREVET EUROPÉEN

(45) Date de publication du fascicule du brevet: **31.05.89**

(51) Int. Cl.⁴: **C 07 C 147/14**, A 61 K 31/16

(21) Numéro de dépôt: **87400114.2**

(22) Date de dépôt: **19.01.87**

(54) **(-)-Benzhydrylsulfinylacétamide, procédé de préparation et utilisation en thérapeutique.**

(30) Priorité: **31.01.86 FR 8601337**

(43) Date de publication de la demande:
**19.08.87 Bulletin 87/34**

(45) Mention de la délivrance du brevet:
**31.05.89 Bulletin 89/22**

(84) Etats contractants désignés:
**AT BE CH DE ES FR GB GR IT LI LU NL SE**

(56) Documents cité:
**FR-A-2 385 693**

(73) Titulaire: **LABORATOIRE L. LAFON, 19 Avenue du Professeur Cadiot, F-94701 Maisons Alfort (FR)**

(72) Inventeur: **Lafon, Louis, 5, rue de l'Alboni, F-75016 Paris (FR)**

(74) Mandataire: **Clisci, Serge, S.A. FEDIT- LORIOT CONSEILS EN PROPRIETE INDUSTRIELLE 38, avenue Hoche, F-75008 Paris (FR)**

**EP 0 233 106 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

**(-)-Benzhydrylsulfinylacétamide, procédé de préparation et utilisation en thérapeutique.**

La présente invention a trait au dérivé lévogyre de benzhydrylsulfinylacétamide à son procédé de préparation et à son utilisation en thérapeutique notamment en tant que moyen antidépresseur et stimulant du système nerveux central (SNC) utile en particulier dans le traitement de l'hypersomnie eu égard à ses effets éveillants.

On sait que le brevet français No. 7 805 590 (publication No. FR-B-2 385 693) décrit le racémique (±)-benzhydrulsulfinylacétamide, qui a pour numéro de Code: CRL 40 476 et pour formule développée:

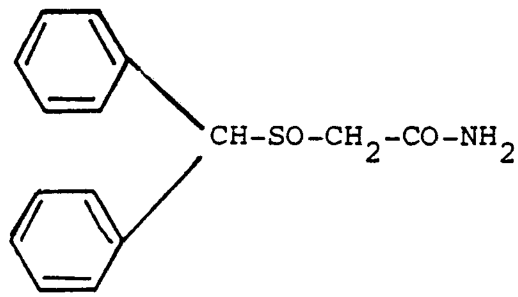

en tant que produit (voir exemple 1 dudit brevet français) et en tant que moyen stimulant du système nerveux central (SNC).

On sait également que dans EP-A-0 097 071 les propriétés neuropsychopharmacologiques du racémique ont été comparées à celles des analogues de formule

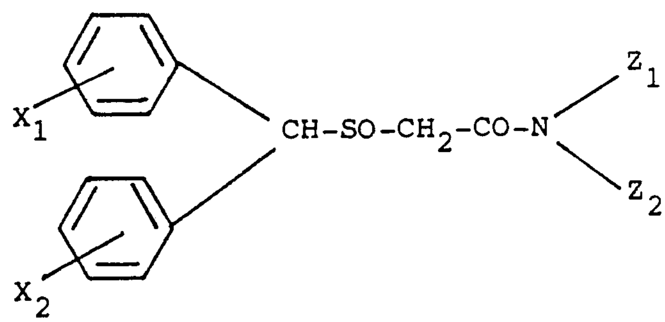

dans laquelle

- $X_1$ et $X_2$, qui peuvent être identiques ou différents, représentent chacun H, Cl ou F,
- $Z_1$ représente $CH_3$, $CH_2CH_3$, $CH(CH_3)_2$ ou $C(CH_3)_3$, $Z_1$ pouvant représenter en outre un atome d'hydrogène quand au moins un des $X_1$ et $X_2$ est différent de H,
- $Z_2$ représente H; $NZ_1Z_2$ considérés ensemble peuvent représenter un groupe pipéridino ou morpholino.

qui agissent sur le SNC, les uns comme sédatifs, les autres comme stimulants (voir notamment le tableau I page 3 et le tableau IV page 4 dudit document européen).

On vient de trouver à présent que le composé lévogyre (-)-benzhydrylsulfinylacétamide (No. de Code: CRL 40 982) présente des propriétés thérapeutiques intéressantes par rapport au racémique (±)-benzhydrylsulfinylacétamide (No. de Code: CRL 40 476) et au composé dextrogyre (+)-benzhydrylsulfinylacétamide (No. de Code: CRL 40 983). De façon surprenante on a constaté que le composé lévogyre a dans l'organisme un métabolisme différent du racémique et du dextrogyre, et qu'il est particulièrement intéressant dans le traitement de l'hypersomnie, et de la maladie d'Alzheimer.

Selon l'invention on préconise donc en tant que produit industriel nouveau, utile en thérapeutique et appartenant à la famille des dérivés benzhydrylsulfinyle, qui est caractérisé par le fait qu'il s'agit du (-)-benzhydrylsulfinylacétamide.

Ce composé lévogyre ne peut pas être préparé par isolation à partir de l'amide racémique correspondant. Cependant on peut le préparer par synthèse chimique à partir d'un précurseur de l'amide, selon une méthode connue en soi par applications de mécanismes réactionnels classique.

Le procédé de préparation que l'on préconise selon l'invention consiste

1°) à faire réagir l'acide (±)-benzhydrylsulfinylacétique avec la (-)-α-méthylbenzylamine pour obtenir le (-)-benzhydrylsulfinylacétate de (-)-α-méthylbenzylamine (de façon avantageuse on opère en présence d'un faible excès d'amine par rapport aux conditions stoechiométriques, et plus particulièrement avec un rapport molaire amine-acide compris entre 1,02/1 et 1,15/1, et mieux entre 1,05/1 et 1,10/1);

2°) à transformer le sel (-)-benzhydrylsulfinylacétate de (-)-α-méthylbenzylamine ainsi obtenu en acide (-)-benzhydrylsulfinylacétique [de façon avantageuse, on procède à une hydrolyse en milieu acide, le solvant étant de l'eau tiède (notamment de l'eau à 30 - 45°C)], et

3°) à soumettre l'acide (-)-benzhydrylsulfinylacétique ainsi obtenu à une réaction d'amidification au moyen d'ammoniac.

De façon avantageuse l'amidification du stade 3°) est effectuée en 2 étapes, à savoir:

3a) estérification de l'acide (-)-benzhydrylsulfinylacétique en (-)-benzhydrylsulfinylacétate d'alkyle inférieur [en $C_1$-$C_3$, notamment d'isopropyle, d'éthyle ou de méthyle (de préference d'éthyle et mieux de méthyle)]; puis

3b) transamidification du (-)-benzhydrylsulfinylacétate d'alkyle inférieur ainsi obtenu au moyen de $NH_3$ (la réaction de transamidification est de préference réalisée dans un alcool inférieur, et mieux au sein de l'alcool correspondant au groupe alkyle de l'ester obtenu au stade 3a), en faisant passer dans le milieu réactionnel un courant de $NH_3$).

L'acide (±)-benzhydrylsulfinylacétique est une substance connue qui est décrite comme intermediaire de synthèse dans FR-B-2 326 181 ($F_{inst.}$ = 164 - 165°C).

Selon l'invention, on préconise une composition thérapeutique qui est caractérisée en ce qu'elle renferme, en association avec un excipient physiologiquement acceptable, le (-)-benzhydrylsulfinylacétamide en tant qu'ingrédient actif. Bien entendu dans une telle composition ledit (-)-benzhydrylsulfinylacétamide intervient en quantité pharmaceutiquement efficace.

On préconise également l'utilisation de ce composé lévogyre pour l'obtention d'un médicament éveillant destiné à une utilisation en thérapeutique humaine vis-à-vis de l'hypersomnie, d'une part, et d'un médicament stimulant et en particulier anti-aphasie et anti-apraxie idéomotrice destiné à une utilisation en thérapeutique vis-à-vis de la maladie d'Alzheimer, d'autre part.

D'autres avantages et caractéristiques de l'invention seront mieux compris à la lecture qui va suivre (i) d'exemples de préparation, et (ii) de résultats d'essais neuropsychopharmacologiques comparatifs. Ces éléments, nullement limitatifs, sont donnés à titre d'illustration.

**Préparation I**

Obtention du (-)-benzhydrylsulfinylacétamide.
(Exemple 1; No. de Code: CRL 40 982).

a) (-)-Benzhydrylsulfinylacétate de (-)-α-méthylbenzylamine.

Dans une suspension de 27,4 g (0,1 mole) d'acide (±)-benzhydrylsulfinylacétique ($F_{inst.}$ = 164 - 165°C; No. de Code: CRL 40 467) dans 500 ml d'eau, on ajoute 13 g (0,108 mole) de (-)-α-méthylbenzylamine; on filtre à chaud, refroidit, essore et recristallise 2 fois dans 300 ml d'eau. On obtient 17 g (rendement 42%) de (-)-benzhydrylsulfinylacétate de (-)-α-méthylbenzylamine.

$F_{inst.}$ = 148 - 150°C.

b) Acide (-)-benzhydrylsulfinylacétique.

Le (-)-benzhydrylsulfinylacétate de (-)-α-méthylbenzylamine (17 g) ainsi obtenu est mis en solution dans 800 ml d'eau tiède (30 - 40°C) puis acidifié avec 7 ml d'acide chlorhydrique concentré (HCl 12N, d = 1,19 g/l). On filtre à froid, lave le précipité avec de l'eau, sèche et obtient l'acide (-)-benzhydrylsulfinylacétique attendu avec un rendement d'environ 100 %.

$F_{inst.}$ = 185 - 188°C.

$\alpha_D^{20°C}$ = -35°C (en solution à 1 % dans $CH_3OH$).

c) (-)-Benzhydrylsulfinylacétate de méthyle.

16,45 g (0,06 mole) d'acide (-)-benzhydrylsulfinylacétique en suspension dans 300 ml d'eau, sont traités à 20°C, sous agitation, avec 16,8 g (0,2 mole) de bicarbonate de sodium et 18,8 ml (0,21 mole) de sulfate de méthyle, on agite 16 à 18 heures à 20°C, filtre, lave à l'eau et sèche. On obtient le (-)-benzhydrylsulfinylacétate de méthyle avec un rendement de 85 %.

$F_{inst.}$ = 109 - 110°C

$\alpha_D^{20°C}$ = -22,5° (en solution à 4 % dans $CH_3OH$).

d) CRL 40 982.

Dans une solution de 100 ml de méthanol renfermant 8,6 g (0,03 mole) de (-)-benzhydrylsulfinylacétate de méthyle on fait passer un courant de $NH_3$ gazeux et sec, à la température ambiante. On fait réagir $NH_3$ ainsi introduit sous agitation pendant 5 h. On évapore le méthanol, reprend le résidu d'évaporation à l'éther, essore et recristallise dans l'éthanol. On obtient le CRL 40982 avec un rendement global de 32

%. Ce produit se présente sous la forme de cristaux blancs, solubles dans les alcools et l'acétone et insolubles dans l'eau et l'éther.
$F_{inst.}$ = 153 - 154°C.
$\alpha_D^{20°C}$ = -20° (en solution à 2 dans $CH_3OH$).

**Préparation II**

Obtention du (+)-benzhydrylsulfinylacétamide.
(Produit de comparaison CP1; No. de Code = CRL 40 983).

En procédant comme indiqué dans la Préparation I ci-dessus mais en remplaçant la (-)-α-méthylbenzylamine par la (+)-α-méthylbenzylamine on obtient successivement:

a) le (+)-benzhydrylsulfinylacétate de (+)-α-méthylbenzylamine;
$F_{inst.}$ = 148 - 150°C;

b) l'acide (+)-benzhydrylsulfinylacétique;
$F_{inst.}$ = 190 - 191°C,
$\alpha_D^{20°C}$ = +45° (en solution à 1 % dans $CH_3OH$);

c) le (+)-benzhydrylsulfinylacétate de méthyle;
$F_{inst.}$ = 109 - 110°C,
$\alpha_D^{20°C}$ = +22,2° (en solution à 4 dans $CH_3OH$);
puis

d) le CRL 40 983;
$F_{inst.}$ = 153 - 154°C.
$\alpha_D^{20°C}$ = +22° (en solution à 2 dans $CH_3OH$).

On a résumé ci-après les essais comparatifs qui ont été entrepris avec le dérivé lévogyre selon l'invention (Ex. 1; No. de Code = CRL 40982), le dérivé dextrogyre (CP1; No. de Code = CRL 40 983) et le racémique correspondant (CP2; No. de Code: CRL 40 476). Dans ces essais les 3 produits à étudier ont été administrés, sauf indications contraires, par voie i.p., en suspension dans une solution aqueuse de gomme arabique sous un volume de 20 ml/kg chez la souris mâle et sous un volume de 5 ml/kg chez le rat mâle.

A - **Toxicité**

Chez la souris mâle, par voie intrapéritonéale, on constate que la DL-0 (dose maximale non mortelle) est supérieure ou égale à 512 mg/kg pour le dextrogyre et le racémique, alors que la DL-30 du lévogyre est de l'ordre d'environ 512 mg/kg.

En bref le CRL 40982 est plus toxique que le CRL 40 483 (CP1) et le CRL 40 476 (CP2). Le fait que la toxicité du CRL 40982 soit supérieure à celle des deux autres produits n'est pas gênant dès lors que le lévogyre présente néanmoins une plage utile de concentrations non mortelles suffisamment étendue. Ici le fait que le CRL 40 982 soit plus toxique que les deux autres produits indique qu'il est plus actif.

B - **Comportement chez le rat**

Alors que chez la souris mâle les CRL 40 982, CRL 40 983 et CRL 40 476 présentent des effets stimulants, en revanche chez le rat mâle on constate que les CRL 40 982 et CRL 40 983 n'ont pas d'effets stimulants alors que le racémique (CRL 40 476) (i) est stimulant et (ii) a une action mydriatique à toutes les doses utilisées, les isomères lévogyre et dextrogyre administrés seuls étant dépourvus d'une telle action mydriatique:

- à la dose de 128 mg/kg, le CRL 40 476 provoque une excitation avec une augmentation de la réaction de peur pendant 2 h, une exophtalmie pendant 1 h et une mydriase pendant 1 à 2 h;
- à la dose de 32 mg/kg, le CRL 40 476 provoque une excitation (fugace, pendant 0,5 h) avec augmentation de la réaction de peur pendant 1 h, une exophtalmie pendant 0,5 - 1 h et une mydriase pendant 1 - 2 h;
- à la dose de 8 mg/kg, le CRL 40 476 provoque une exophtalmie pendant 0,5 - 1 h, et une mydriase pendant 0,5 h;
- à la dose de 2 mg/kg, le CRL 40 476 induit une mydriase fugace se manifestant 1 h après administration;
  alors que
- aux doses de 64 mg/kg, 16 mg/kg, 4 mg/kg et 1 mg/kg, les CRL 40 982 et CRL 40 983 ont des comportements, réactivités, variation de la température rectale et du diamètre papillaire sensiblement comparables à ceux du lot-témoin.

C - **Activité motrice chez la souris**

Les souris (6 par dose, 18 témoins) reçoivent le CRL 40 476, le CRL 40 982 ou le CRL 40 983, 4 heures avant d'être placées en actimètre où leur motilité est enregistrée pendant 30 minutes. On constate que les trois substances utilisées entraînent, aux doses de 128 mg/kg et, à un degré moindre, 64 mg/kg une augmentation de l'activité motrice quatre heures après leur administration. Cependant, l'hyperactivité induite par le CRL 40 982 et le CRL 40 983 atteint un niveau et un degré de signification statistique supérieurs à ceux dus au CRL 40 476, notamment à la plus forte dose utilisée (128 mg/kg).

En bref, dans les conditions de l'expérimentation (administration des substances par voie intrapéritonéale quatre heures avant test) le CRL 40 982 montre un effet stimulant d'intensité égale à celle observée avec le CRL 40 983, alors que l'hyperactivité induite par le CRL 40 476 est inférieure à celle obtenue tant avec le CRL 40 982 qu'avec le CRL 40 983.

D - **Etude pharmacocinétique**

Dans l'organisme le CRL 40 476 se transforme en partie en acide (±)-benzhydrylsulfinylacétique (CRL 40 467) de formule développée

$(C_6H_5)_2CH\text{-}SO\text{-}CH_2\text{-}COOH$

qui est utilisé comme matière première pour la synthèse des isomères optiques CRL 40 982 et CRL 40 983.

Or il se trouve que ce métabolite est inactif. En effet le CRL 40 467 administré par voie intrapéritonéale chez la souris mâle aux doses de 1024 mg/kg, 512 mg/kg, 256 mg/kg, 128 mg/kg et 64 mg/kg induit l'apparition d'une sédation brève (d'une durée inférieure ou égale à environ 50 - 60 minutes), et ne provoque aucun décès chez les animaux traités. Son étude neuropsychopharmacologique n'a révélé aucune activité psychotrope.

Pour distinguer le CRL 40 982 des composés racémique et dextrogyre, l'étude cinétique du métabolisme a été entreprise chez le chien (lot de quatre animaux). A raison d'une administration orale par semaine, chaque animal a reçu de façon randomisée et croisée: 2 administrations de CRL 40 476 (pour apprécier la variation intraindividuelle), 1 administration de CRL 40 982 et 1 administration de CRL 40 983. A la suite de chacune de ces administrations, les cinétiques du CRL 40 476 et du CRL 40 467 présents dans le plasma ont été déterminées (sans recherches si ces deux produits sont sous forme racémique, lévogyre et/ou dextrogyre en raison des difficultés de dosage en milieu biologique du pouvoir rotatoire de chacun des isomères optiques).

La dose administrée pour chaque substance CRL 40 476, CRL 40 982 et CRL 40 983 à tester était de 30 mg/kg.

Après administration du CRL 40 476 on retrouve dans le plasma ledit CRL 40 476 et son métabolite, le CRL 40 4467.

Après administration du CRL 40 982 on retrouve dans le plasma ledit CRL 40 982, que l'on va caractériser et doser par commodité, eu égard à ce qui a été indiqué ci-dessus, comme étant du CRL 40 476, et un métabolite que l'on va caractériser et doser comme étant du CRL 40 467.

De même après administration du CRL 40 983 on va caractériser et doser les 2 produits plasmatiques correspondants comme étant les CRL 40 476 et CRL 40 467.

On trace les courbes des concentrations plasmatiques desdits CRL 40 476 et CRL 40 467 en fonction du temps de l'instant T = 0 à l'instant T = +9 h après administration des CRL 40 476, CRL 40 982 et CRL 40 983. On calcule ensuite les aires sous les courbes ($ASC_0{}^{+9}$). Les résultats obtenus sont consignés dans le tableau I ci-après. Ils mettent en évidence que

a) après administration des CRL 40 476 et CRL 40 983, les $ASC_0{}^{+9h}$ de CRL 40 476 ne sont pas statistiquement différentes, alors que, après administration du CRL 40 982, la $ASC_0{}^{+9h}$ de CRL 40 476 est approximativement voisine du double des $ASC_0{}^{+9h}$ de CRL 40 476 qui résultent chacune de l'administration des CRL 40 476 et CRL 40 983; et
b) la quantité de CRL 40 467 produite après administration de CRL 40 983 est très importante (83,12 $mg.1^{-1}.h$) alors que celle produite après administration de CRL 40 982 est très faible (8,69 $mg.1^{-1}.h$).

L'intérêt du CRL 40 982 selon l'invention réside dans le fait que ce produit ne subit qu'une faible transformation en CRL 40 467 inactif alors que le dérivé dextrogyre correspondant est très fortement métabolisé en CRL 40 467. En bref, le composé lévogyre CRL 40 982 présente une meilleure biodisponibilité que le composé racémique CRL 40 476 et le composé dextrogyre CRL 40 983, eu égard à la faible quantité de métabolite inactif qu'il donne dans l'organisme.

Ces résultats pharmacocinétiques ont été confirmés chez le lapin et la souris. On a également observé in vitro un effet immunostimulant pour le CRL 40 982.

E - **Essais cliniques**

En clinique humaine on a constaté que la demivie d'élimination du CRL 40 982 est relativement longue (voisine de 10 h) ce qui permet d'obtenir de bons résultats chez l'adulte avec 1 à 2 prises quotidiennes.

Au cours des essais cliniques, le CRL 40 982 s'est révélé agir, à court terme, en tant que substance anti-éveillante, pure hypnogène, et à long terme, en tant que substance éveillante utile vis-à-vis de l'hypersomnie. Par ailleurs, tant à court terme qu'à long terme le CRL 40 982 s'est avéré être particulièrement actif vis-à-vis des symptômes de démence et de la perte de mémoire (notamment chez le vieillard).

Sous forme de comprimés ou gélules renfermant chacun 50 à 100 mg de CRL 40 982, et à raison d'une à deux prises par jour, ce produit présente un profil neuropsychopharmacologique stimulant, différent des substances amphétaminiques et des antidépresseurs tricycliques, et est utile vis-à-vis des dépressions, de l'hypersomnie et en particulier vis-à-vis de la maladie d'Alzheimer (amélioration des symptômes de démence, des troubles de mémoire, de l'aphasie et de l'apraxie idéomotrice).

**Revendications** pour les Etats Contractants: BE, CH, DE, FR, GB, IT, LI, LU, NL, SE

1.(-)-Benzhydrylsulfinylacétamide.

2. Composition thérapeutique caractérisée en ce qu'elle renferme en association avec un excipient physiologiquement acceptable, une quantité pharmaceutiquement efficace de (-)-benzhydrylsulfinylacétamide.

3. Utilisation du (-)-benzhydrylsulfinylacétamide pour l'obtention d'un médicament éveillant destiné à une utilisation en thérapeutique humaine vis-à-vis de l'hypersomnie.

4. Utilisation du (-)-benzhydrylsulfinylacétamide pour l'obtention d'un médicament stimulant du SNC destiné à une utilisation en thérapeutique humaine vis-à-vis de la maladie d'Alzheimer.

5. Procédé de préparation de (-)-benzhydrylsulfinylacétamide caractérisé en ce que

1°) on fait réagir l'acide (±)-benzhydrylsulfinylacétique avec la (-)-α-méthylbenzylamine pour obtenir le (-)-benzhydrylsulfinylacétate de (-)-α-méthylbenzylamine;

2°) on transforme le sel (-)-benzhydrylsulfinylacétace de (-)-α-méthylbenzylamine ainsi obtenu en acide (-)-benzhydrylsulfinylacétique par hydrolyse acide; et

3°) on soumet l'acide (-)-benzhydrylsulfinylacétique ainsi obtenu à une réaction d'amidification au moyen d'ammoniac.

6. Procédé selon la revendication 5, caractérisé en ce que le stade 3°) est réalisé en deux étapes:

3a) estérification de l'acide (-)-benzhydrylsulfinylacetique en (-)-benzhydrylsulfinylacétate d'alkyle inférieur en $C_1$-$C_3$; puis

3b) transamidification du (-)-benzhydrylsulfinylacétate d'alkyle inférieur ainsi obtenu au moyen de $NH_3$.

7. Procédé selon l'une quelconque des revendications 5 et 6, caractérisé en ce que

- au stade 1°) on opère en présence d'un excès d'amine par rapport aux conditions stoechiométriques avec un rapport molaire amine-acide compris entre 1,02/l et 1,15/l;
- au stade 2°) l'hydrolyse acide est effectuée à une température comprise entre 30 et 45°C;
- à l'étape 3a) la réaction d'estérification est réalisée de façon à obtenir un ester d'alkyle inférieur en $C_1$-$C_3$ choisi parmi les esters d'isopropyle, d'éthyle et de méthyle; et,
- à l'étape 3b) la réaction de transamidification est réalisée au moyen d'un courant gazeux de $NH_3$.

**Revendications** pour les Etats Contractants: AT, ES, GR

1. Procédé de préparation de (-)-benzhydrylsulfinylacétamide caractérisé en ce que

1°) on fait réagir l'acide (±)-benzhydrylsulfinylacétique avec la (-)-α-méthylbenzylamine pour obtenir le (-)-benzhydrylsulfinylacétate de (-)-α-méthylbenzylamine;

2°) on transforme le sel (-)-benzhydrylsulfinylacétace de (-)-α-méthylbenzylamine ainsi obtenu en acide (-)-benzhydrylsulfinylacétique par hydrolyse acide; et

3°) on soumet l'acide (-)-benzhydrylsulfinylacétique ainsi obtenu à une réaction d'amidification au moyen d'ammoniac.

2. Procédé selon la revendication 1, caractérisé en ce que le stade 3°) est réalisé en deux étapes:

3a) estérification de l'acide (-)-benzhydrylsulfinylacetique en (-)-benzhydrylsulfinylacétate d'alkyle inférieur en $C_1$-$C_3$; puis

3b) transamidification du (-)-benzhydrylsulfinylacétate d'alkyle inférieur ainsi obtenu au moyen de $NH_3$.

3. Procédé selon l'une quelconque des revendications 1 et 2, caractérisé en ce que

- au stade 1°) on opère en présence d'un excès d'amine par rapport aux conditions stoechiométriques avec un rapport molaire amine-acide compris entre 1,02/l et 1,15/l;
- au stade 2°) l'hydrolyse acide est effectuée à une température comprise entre 30 et 45°C;
- à l'étape 3a) la réaction d'estérification est réalisée de façon à obtenir un ester d'alkyle inférieur en $C_1$-$C_3$ choisi parmi les esters d'isopropyle, d'éthyle et de méthyle; et,
- à l'étape 3b) la réaction de transamidification est réalisée au moyen d'un courant gazeux de $NH_3$.

**Patentansprüche** für die Vertragsstaaten: BE, CH, DE, FR, GB, IT, LI, LU, NL, SE

1. (-)-Benzhydrylsulfinylacetamid.

2. Therapeutische Zusammensetzung, dadurch gekennzeichnet, daß sie, zusammen mit einem physiologisch annehmbaren Träger, eine pharmazeutisch wirksame Menge an (-)-Benzhydrylsulfinylacetamid enthält.

3. Verwendung von (-)-Benzhydrylsulfinylacetamid zur Herstellung eines erregenden Medikamentes zur Verwendung in der Humantherapie der Hypersomnie.

4. Verwendung von (-)-Benzhydrylsulfinylacetamid zur Herstellung eines das ZNS stimulierenden Medikamentes zur Verwendung in der Humantherapie der Alzheimer-Krankheit.

5. Verfahren zur Herstellung von (-)-Benzhydrylsulfinylacetamid, dadurch gekennzeichnet, daß man

1) (±)-Benzhydrylsulfinylessigsäure mit (-)-α-Methylbenzylamin zum (-)-α-Methylbenzylamin-(-)-benzhydrylsulfinylacetat umsetzt;

2) das so erhaltene (-)-α-Methylbenzylamin-(-)-benzhydrylsulfinylacetat-Salz durch saure Hydrolyse in die (-)-Benzhydrylsulfinylessigsäure überführt;

und

3) die so erhaltene (-)-Benzhydrylsulfinylessigsäure einer Amidierung mit Ammoniak unterwirft.

6. Verfahren gemäß Anspruch 5, dadurch gekennzeichnet, daß man die Stufe 3 in zwei Schritten durchführt:

3a) Veresterung der (-)-Benzhydrylsulfinylessigsäure zum (-)-Benzhydrylsulfinylessigsäure-$C_1$-$C_3$-Niederalkylester; und dann

3b) Transamidierung des so erhaltenen (-)-Benzhydrylsulfinylessigsäure-Niederalkylesters mit Ammoniak.

7. Verfahren nach einem der Ansprüche 5 und 6, dadurch gekennzeichnet, daß man

- in Stufe in Gegenwart eines Überschusses (in bezug auf stöchiometrische Verhältnisse) an Amin mit einem Molverhältnis Amin-Säure von zwischen 1,02/l und 1,15/l arbeitet;
- in Stufe 2 die saure Hydrolyse bei einer Temperatur zwischen 30 und 45°C durchführt;
- in Stufe 3a die Veresterung so durchführt, daß man einen $C_1$-$C_3$-Niederalkylester erhält, der ausgewählt ist aus Isopropyl-, Ethyl- und Methylester;

und

- in der Stufe 3b die Transamidierung mit Hilfe eines Ammoniakgasstroms durchführt.

**Patentansprüche** für die Vertragsstaaten: AT, ES, GR

1. Verfahren zur Herstellung von (-)-Benzhydrylsulfinylacetamid, dadurch gekennzeichnet, daß man

1) (±)-Benzhydrylsulfinylessigsäure mit (-)-α-Methylbenzylamin zum (-)-α-Methylbenzylamin-(-)-benzhydrylsulfinylacetat umsetzt;

2) das so erhaltene (-)-α-Methylbenzylamin-(-)-benzhydrylsulfinylacetat-Salz durch saure Hydrolyse in die (-)-Benzhydrylsulfinylessigsäure überführt;

und

3) die so erhaltene (-)-Benzhydrylsulfinylessigsäure einer Amidierung mit Ammoniak unterwirft.

2. Verfahren gemäß Anspruch 1, dadurch gekennzeichnet, daß man die Stufe 3 in zwei Schritten durchführt:

3a) Veresterung der (-)-Benzhydrylsulfinylessigsäure zum (-)-Benzhydrylsulfinylessigsäure-$C_1$-$C_3$-Niederalkylester; und dann

3b) Transamidierung des so erhaltenen (-)-Benzhydrylsulfinylessigsäure-Niederalkylesters mit Ammoniak.

3. Verfahren nach einem der Ansprüche 1 und 2, dadurch gekennzeichnet, daß man

- in Stufe in Gegenwart eines Überschusses (in bezug auf stöchiometrische Verhältnisse) an Amin mit einem Molverhältnis Amin-Säure von zwischen 1,02/l und 1,15/l arbeitet;
- in Stufe 2 die saure Hydrolyse bei einer Temperatur zwischen 30 und 45°C durchführt;
- in Stufe 3a die Veresterung so durchführt, daß man einen $C_1$-$C_3$-Niederalkylester erhält, der ausgewählt ist aus Isopropyl-, Ethyl- und Methylester;

und

- in der Stufe 3b die Transamidierung mit Hilfe eines Ammoniakgasstroms durchführt.

**Claims** for the Contracting States: BE, CH, DE, FR, GB, IT, LI, LU, NL, SE

1. (-)-Benzhydrylsulfinylacetamide.

2. A therapeutic composition comprising a pharmaceutically effective amount of (-)-benzhydrylsulfinyl acetamide, in association with a physiologically acceptable excipient.

3. The use of (-)-benzhydrylsulfinylacetamide to obtain an arousing drug to be used in human therapy for hypersomnia.

4. The use of (-)-benzhydrylsulfinylacetamide to obtain a CNS-stimulating drug to be used in human therapy for Alzheimer's disease.

5. A method for the preparation of (-)-benzhydrylsulfinylacetamide, which comprises:

1°)reacting (±)-benzhydrylsulfinylacetic acid with (-)-α-methylbenzylamine to give the (-)-benzhydrylsulfinylacetate of (-)-α-methylbenzylamine,

2°) converting the resulting (-)-benzhydrylsulfinylacetate salt of (-)-α-methylbenzylamine to benzhydrylsulfinylacetic acid by acid hydrolysis, and

3°) subjecting the resulting (-)-benzhydrylsulfinylacetic acid to an amidation reaction with $NH_3$.

6. The method according to claim 5, wherein stage 3°) is carried out in two steps, namely:

3a) esterification of the (-)-benzhydrylsulfinylacetic acid to a $C_1$-$C_3$ lower alkyl (-)-benzhydrylsulfinylacetate, followed by

3b) transamidation of the resulting $C_1$-$C_3$ lower alkyl (-)-benzhydrylsulfinylacetate with $NH_3$.

7. The method according to claim 5 or 6, wherein:

- in stage 1°), the reaction is carried out in the presence of an excess of amine relative to the stoichiometric conditions, with a molar ratio amine/acid of between 1.02/l and 1.15/l,

- in stage 2°), the said acid hydrolysis is carried out at a temperature of between 30 and 45°C,

- in step 3a), the said esterification reaction is carried out so as to give a $C_1$-$C_3$ lower alkyl ester selected from the group comprising the isopropyl, ethyl and methyl esters, and

- in step 3b), the said transamidation reaction is carried out with a stream of $NH_3$ gas.

**Claims** for the Contracting States: AT, ES, GR

1. A method for the preparation of (-)-benzhydrylsulfinylacetamide, which comprises:

1°)reacting (±)-benzhydrylsulfinylacetic acid with (-)-α-methylbenzylamine to give the (-)-benzhydrylsulfinylacetate of (-)-α-methylbenzylamine,

2°) converting the resulting (-)-benzhydrylsulfinylacetate salt of (-)-α-methylbenzylamine to (-)-benzhydrylsulfinylacetic acid by acid hydrolysis, and

3°) subjecting the resulting (-)-benzhydrylsulfinylacetic acid to an amidation reaction with $NH_3$.

2. The method according to claim 1, wherein stage 3°) is carried out in two steps, namely:

3a) esterification of the (-)-benzhydrylsulfinylacetic acid to a $C_1$-$C_3$ lower alkyl (-)-benzhydrylsulfinylacetate, followed by

3b) transamidation of the resulting $C_1$-$C_3$ lower alkyl (-)-benzhydrylsulfinylacetate with $NH_3$.

3. The method according to claim 1 or 2, wherein:

- in stage 1°), the reaction is carried out in the presence of an excess of amine relative to the stoichiometric conditions, with a molar ratio amine/acid of between 1.02/l and 1.15/l,

- in stage 2°), the said acid hydrolysis is carried out at a temperature of between 30 and 45°C,

- in step 3a), the said esterification reaction is carried out so as to give a $C_1$-$C_3$ lower alkyl ester selected from the group comprising the isopropyl, ethyl and methyl esters, and

- in step 3b), the said transamidation reaction is carried out with a stream of $NH_3$ gas.